# EUROPÄISCHE PATENTANMELDUNG

(11) **EP 2 036 517 A1**
(43) Veröffentlichungstag der Anmeldung: **18.03.2009**
(21) Anmeldenummer: 07018225.8
(22) Anmeldetag: 17.09.2007
(51) Int. Cl.: A61F 2/30, A61L 27/28

(54) **Endoprothesenkomponente**

(71) Anmelder: WALDEMAR LINK GmbH & Co. KG, 22339 Hamburg (DE)
(72) Erfinder: Thull, Roger, Prof., Dr., 97082 Würzburg (DE); Link, Helmut D., 22397 Hamburg (DE)
(74) Vertreter: Riesenberg, Axel

(57) **Zusammenfassung**

Die Erfindung betrifft eine Endoprothesenkomponente (17) sowie ein Verfahren zum Herstellen einer Endoprothesenkomponente (17). Die Endoprothesenkomponente (17) umfasst einen die Form der Endoprothesenkomponente (17) vorgebenden Körper (23). Der Körper (23) ist auf Oberflächenanteilen (22), mit denen die Endoprothesenkomponente im implantierten Zustand in Kontakt mit menschlichem Gewebe steht, mit einer Außenschicht (25) belegt ist, die ein Nitrid, ein O-xinitrid oder ein Oxid auf der Basis eines Refraktärmetalls umfasst. Die Endoprothesenkomponente (17) zeichnet sich erfindungsgemäß dadurch aus, dass in der Außenschicht (25) Silber und/oder Kupfer enthalten ist. Mit dem erfindungsgemäßen Verfahren wird die Außenschicht (25) aufgebracht. Die erfindungsgemäße Außenschicht hat den Vorteil, dass sie gute mechanische Eigenschaften mit einer antimikrobiellen Wirkung verbindet.

## Beschreibung

Die Erfindung betrifft eine Endoprothesenkomponente mit einem die Form der Endoprothesenkomponente vorgebenden Körper. Auf Oberflächenanteilen, mit denen die Endoprothesenkomponente im implantierten Zustand Kontakt mit menschlichem Gewebe eingeht, ist der Körper mit einer Außenschicht belegt, die ein Nitrid, ein Oxinitrid oder ein Oxid auf der Basis eines Refraktärmetalls umfasst. Die Erfindung betrifft ferner ein Verfahren zur Herstellung einer solchen Endoprothesenkomponente.

Endoprothesenkomponenten, die Bestandteile des menschlichen Skeletts ersetzen, müssen mehrere Anforderungen erfüllen. Die Endoprothesenkomponente muss hinreichend stabil sein, damit sie die im Skelett auftretenden Kräfte aufnehmen kann. Außerdem müssen die Elemente der Endoprothesenkomponente, die in implantiertem Zustand Kontakt mit Gewebe, was auch Knochenmaterial umfasst, des menschlichen Körpers eingehen, eine gute Verträglichkeit aufweisen.

Bereits bekannt ist es, Oberflächenanteile der Endoprothesenkomponente, die für Kontakt mit menschlichem Gewebe ausgelegt sind, mit einer Schicht aus einem harten Material, wie beispielsweise einem Nitrid, einem Oxinitrid oder einem Oxid auf der Basis eines Refraktärmetalls zu belegen. Beschichtungen dieser Art verbinden gute mechanische Eigenschaften mit einer guten Verträglichkeit.

Dennoch kommt es bei der Annahme derartiger Endoprothesenkomponente durch den menschlichen Körper gelegentlich zu Problemen. Es kann nämlich passieren, dass sich auf der Oberfläche der Endoprothesenkomponente Mikroorganismen festsetzen, die die Aufnahme der Endoprothesenkomponente in den menschlichen Körper behindern. Die Gefahr durch Mikroorganismen auf der Oberfläche von Endoprothesenkomponenten ist je größer, desto größer der Eingriff ist, der zum Einsetzen der Endoprothesenkomponente in den Körper erforderlich ist, und je größer die Fläche ist, über die die Endoprothesenkomponente mit dem Gewebe des Körpers in Berührung kommt. Operationen, bei denen ein großer Eingriff erforderlich ist und bei denen die Endoprothese großflächig mit Gewebe des menschlichen Körpers in Kontakt gebracht wird, sind beispielsweise Kopf-Schaft-Prothesenschäfte oder solche, bei denen der Oberschenkelknochen komplett durch eine Endoprothese ersetzt wird.

Der Erfindung liegt die Aufgabe zu Grunde, eine Endoprothesenkomponente der eingangs genannten Art sowie ein Verfahren zur Herstellung einer solchen Prothese vorzustellen, bei denen die Wahrscheinlichkeit, dass die Aufnahme der Endoprothesenkomponente in den menschlichen Körper durch Mikroorganismen behindert wird, vermindert ist. Die Aufgabe wird gelöst durch die Merkmale der Ansprüche 1 und 7. Vorteilhafte Ausführungsformen finden sich in den Unteransprüchen. Die erfindungsgemäße Endoprothesenkomponente zeichnet sich dadurch aus, dass die Außenschicht Silber und/oder Kupfer enthält.

Zunächst werden einige Begriffe erläutert. Als Außenschicht wird eine Schicht bezeichnet, die unmittelbaren Kontakt zur Umgebung hat. Eine Außenschicht ist also nicht mit einer weiteren Schicht belegt.

Ein Körper, der die Form der Endoprothesenkomponente vorgibt, unterscheidet sich zumindest in dem Bereich, in dem die Oberfläche der Endoprothesenkomponente für einen Kontakt mit menschlichem Gewebe ausgelegt ist, nur durch zusätzlich auf die Oberfläche aufgebrachte Beschichtungen von der Form der Endoprothesenkomponente.

Regelmäßig ist nicht die gesamte Oberfläche der Endoprothesenkomponente dazu bestimmt, eine Verbindung mit menschlichem Gewebe einzugehen. Vielmehr kann die Endoprothesenkomponente Bereiche an ihrer Oberfläche umfassen, die dazu ausgelegt sind, beispielsweise mit einer weiteren Endoprothesenkomponente zusammenzuwirken, etwa um ein Gelenk zu bilden. Im Rahmen der Erfindung ist es nicht erforderlich, dass der gesamte Bereich der Oberfläche, der für Kontakt mit menschlichem Gewebe ausgelegt ist, mit einer Außenschicht belegt ist. Ausreichend kann es vielmehr, wenn Oberflächenanteile dieser Bereiche mit einer Außenschicht belegt sind.

Der Begriff menschliches Gewebe ist umfassend zu verstehen. Er umfasst alle Elemente des menschlichen Körpers, mit denen eine Endoprothese in Berührung kommen kann, also beispielsweise auch Knochen.

Refraktärmetalle sind hochschmelzende, unedle Metalle der vierten, fünften und sechsten Nebengruppe. Umfasst sind in der vierten Nebengruppe Titan, Zirconium und Hafnium, in der fünften Nebengruppe Vanadium, Niob und Tantal sowie in der sechsten Nebengruppe Chrom, Molybdän und Wolfram. Für die Beschichtung von Endoprothesenkomponenten besonders geeignete Refraktärmetalle sind Titan, Zirkonium, Niob und Tantal. Als Nitrid, Oxinitrid oder Oxid auf der Basis eines Refraktärmetalls werden Verbindungen bezeichnet, die Ionen eines Refraktärmetalls mit Sauerstoff und/oder Stickstoff als reaktivem Gas eingehen. Diese Verbindungen zeichnen sich durch eine große Härte aus.

Wenn die Außenschicht zusätzlich Silber und/oder Kupfer umfasst, so sind bei der Entstehung des Nitrids, Oxinitrids bzw. Oxids nicht nur Ionen des Refraktärmetalls und ein reaktives Gas, sondern zusätzlich Silber- und/oder Kupferionen beteiligt. Die Silber- und/oder Kupferionen werden in die entstehende Beschichtung integriert.

Es hat sich gezeigt, dass die Außenschicht durch das Hinzufügen von Silber und/oder Kupfer eine antimikrobielle Wirkung erhält. Die Verminderung von Mikroorganismen auf der Oberfläche der Endoprothesenkomponente führt dazu, dass die Endoprothesenkomponente besser vom Körper aufgenommen wird. Die erfindungsgemäße Außenschicht verbindet die antimikrobielle Wirkung mit guten mechanischen Eigenschaften wie großer Härte und hoher Abriebfestigkeit. Durch die guten mechanischen Eigenschaften hebt sich die erfindungsgemäße Außenschicht von bekannten Beschichtungen mit antimikrobieller Wirkung ab.

Der Körper, der die Form der Endoprothesenkomponente vorgibt, kann aus Metall oder aus einer Metalllegierung bestehen. Besonders geeignete Materialien sind Titan und Titanlegierungen. Möglich ist es, dass die Außenschicht unmittelbar auf den Körper aufgebracht ist, wobei von dem Begriff Außenschicht auch eine Schicht umfasst ist, die in mehreren Auftragdurchgängen erzeugt wurde. Das Aufbringen einer Schicht in mehreren Auftragdurchgängen kommt insbesondere dann in Betracht, wenn sich in einem Auftragdurchgang keine flächendeckende Schicht erzeugen lässt.

Es kann aber auch eine Zwischenschicht zwischen der Außenschicht und dem Körper vorgesehen sein. Die Zwischenschicht kann eine Schicht eines Nitrids, eines Oxinitrids oder eines Oxids auf der Basis eines Refraktärmetalls sein. Es ist nicht erforderlich, dass die Zwischenschicht Silber oder Kupfer umfasst. Es kann ausreichen, wenn Silber und/oder Kupfer nur in der Außenschicht vorhanden ist, selbst wenn die Außenschicht für sich genommen nicht deckend wäre.

Die Zwischenschicht kann aber auch eine Schicht aus Silber, Kupfer und/oder Gold sein. Vorzugsweise ist die Zwischenschicht in diesem Fall flächendeckend. Eine Zwischenschicht dieser Art kann die antimikrobielle Wirkung der Außenschicht unterstützen. Vorzugsweise ist die auf der Zwischenschicht aufliegende Außenschicht dazu so ausgebildet, dass trotz der Außenschicht ein Kontakt zwischen den Mikroorganismen und der Zwischenschicht möglich ist. Möglich ist ein solcher Kontakt beispielsweise, wenn die Außenschicht porös ist. Die Außenschicht kann auch mit makroskopischen Durchbrechungen versehen sein, die einen größerflächigen Kontakt zur Zwischenschicht ermöglichen. Insbesondere kann die Außenschicht so gestaltet sein, dass die Durchbrechungen einen Flächenanteil von mindestens 20%, vorzugsweise mindestens 40%, weiter vorzugsweise mindestens 60% einnehmen. Eine Außenschicht, die keinen Kontakt zu einer darunter liegenden Schicht ermöglicht, wird als dicht bezeichnet. Auch eine dichte Außenschicht ist von der Erfindung umfasst.

Die antimikrobielle Wirkung kann verstärkt werden, wenn die Außenschicht eine Mikro- oder Nanostruktur mit ausgeprägten Spitzen hat. Eine ausgeprägte Spitze in einer Oberflächenstruktur bezeichnet eine Formation, bei der die Oberfläche ausgehend von einem Extremum in allen Richtungen mit einem Winkel von mehr als 45° bezogen auf die Richtung der Oberfläche abfällt. Elektrisch leitfähige Spitzen bilden ein im Vergleich zu glatten Oberflächen starkes elektrisches Feld in ihrer Umgebung aus, das eine negative Wirkung auf Mikroorganismen ausübt.

Die Erfindung betrifft ferner ein Verfahren zum Herstellen einer solchen Endoprothesenkomponente. Zum Herstellen der Endoprothesenkomponente wird zunächst ein Körper bereitgestellt, der die Form der Endoprothesenkomponente vorgibt. An die Gestalt der Oberfläche des Körpers werden keine besonderen Anforderungen gestellt. Die Oberfläche kann also glatt, makro-, mikro- oder nanostrukturiert sein.

Ferner werden Targets bereitgestellt, aus denen das Material, das später die Beschichtung bilden soll, herausgelöst werden kann. Es kann ein einzelnes Target bereitgestellt werden, das sowohl das Refraktärmetall als auch das Silber und/oder Kupfer umfasst. Möglich ist es aber auch, mehrere Targets bereitzustellen, wobei ein erstes Target ein Refraktärmetall umfasst und wobei ein zweites Target Silber und/oder Kupfer umfasst.

Aus dem einen oder den mehreren Targets werden Ionen des Refraktärmetalls sowie Ionen von Silber und/oder Kupfer herausgelöst. Zum Herauslösen der Ionen kann beispielsweise ein Lichtbogen zwischen einer Elektrode und dem Target erzeugt werden, der dem Target lokal begrenzt soviel Energie zuführt, dass Ionen herausgelöst werden. Eine andere Möglichkeit, dem Target lokal ausreichend Energie zuzuführen, kann darin bestehen, einen Laserstrahl auf das Target zu richten. Alternativ, wenn auch sehr teuer, ist die Energiezufuhr auch durch einen Elektronenstrahl möglich.

Die freien Ionen werden auf den Körper geleitet. Dazu kann eine Spannung zwischen das Target und den Körper gelegt werden, durch die die Ionen in Richtung des Körpers beschleunigt werden. Das Reaktionsgefäß mit Target und Körper enthält ein reaktives Gas, wie beispielsweise Sauerstoff oder Stickstoff oder ein Gemisch von Sauerstoff und Stickstoff. Die Ionen des Refraktärmetalls sowie die Ionen von Silber und/oder Kupfer reagieren mit dem reaktiven Gas. Durch die auf der Oberfläche des Körpers stattfindende Reaktion bildet sich die erfindungsgemäße Außenschicht. Die Reaktion kann unter Vakuum, vorzugsweise unter Hochvakuum stattfinden. Verfahren dieser Art sind unter der Bezeichnung Plasma-Beschichtungsverfahren bekannt. Ein verbesserter Halt einer aufgebrachten Schicht kann erreicht werden, wenn die Oberfläche des Körpers zuvor durch Sandstrahlen behandelt wird.

Werden die Parameter des Plasma-Beschichtungsverfahrens geeignet gewählt, so entsteht eine Außenschicht, die eine Mikro- oder Nanostruktur mit ausgeprägten Spitzen aufweist.

Vor dem Aufbringen der Außenschicht kann der Körper mit einer Zwischenschicht aus Silber und/oder Kupfer versehen werden. Die Zwischenschicht kann ebenfalls mittels eines Plasma-Beschichtungsverfahrens aufgebracht werden. Alternativ ist es möglich, die Zwischenschicht mittels elektrochemischer Abscheidung auf den Körper aufzubringen. Damit die Zwischenschicht ihre antimikrobielle Wirkung entfalten kann, muss sie durch die Außenschicht hindurch zugänglich sein. Eine poröse Außenschicht kann beispielsweise dadurch erzeugt werden, dass dem einzelnen oder den mehreren Targets soviel Energie zugeführt wird, dass sich außer den Ionen auch noch Bruchstücke aus den Targets herauszulösen, die einen größten Durchmesser von mehr als 20 *µ*m haben. Die Bruchstücke bewegen sich statistisch in alle Richtungen und treffen dabei auch auf die Oberfläche des Körpers auf, auf der sich gerade die Außenschicht bildet. An den Stellen, an denen die Bruchstücke auftreffen, wird ein Stück aus der sich bildenden Außenschicht herausgeschlagen. Es entsteht eine poröse Außenschicht, durch die hindurch die Zwischenschicht zugänglich ist.

Eine andere Möglichkeit, die Zugänglichkeit der Zwischenschicht zu gewährleisten, besteht darin, den zu beschichtenden Oberflächenanteil des Körpers mit einer Maske aus einem temperaturstabilen Material zu belegen. Die Maske belegt Teile der Oberfläche und lässt andere Teile der Oberfläche frei. Die Maske kann beispielsweise eine netzartige oder eine gitterartige Form haben. Die Außenschicht bildet sich dann inselförmig nur an den Stellen aus, die von der Maske freigelassen werden. Nach dem Entfernen des temperaturstabilen Materials wird die Zwischenschicht entsprechend der Maske zugänglich. Als temperaturstabil wird ein Material bezeichnet, das gegenüber den bei Plasma-Beschichtungsverfahren bestehenden Bedingungen beständig ist. Das temperaturstabile Material kann insbesondere ein Metall sein.

Die Erfindung wird nachfolgend unter Bezugnahme auf die beigefügten Zeichnungen anhand einer vorteilhaften Ausführungsform beispielhaft beschrieben. Es zeigen:
- Fig. 1: eine mehrere Endoprothesenkomponenten umfassende Endoprothese;
- Fig. 2: eine erfindungsgemäße Endoprothesenkomponente;
- Fig. 3: ein vergrößerter Ausschnitt aus einem Querschnitt durch Fig. 2;
- Fig. 4: die Ansicht aus Fig. 3 bei einer anderen Ausführungsform der Erfindung;
- Fig. 5: die Ansicht aus Fig. 3 bei einer weiteren Ausführungsform der Erfindung; und
- Fig. 6: ein vergrößerter Ausschnitt aus der Oberfläche der Endoprothesenkomponente gemäß Fig. 2 bei noch einer weiteren Ausführungsform der Erfindung.

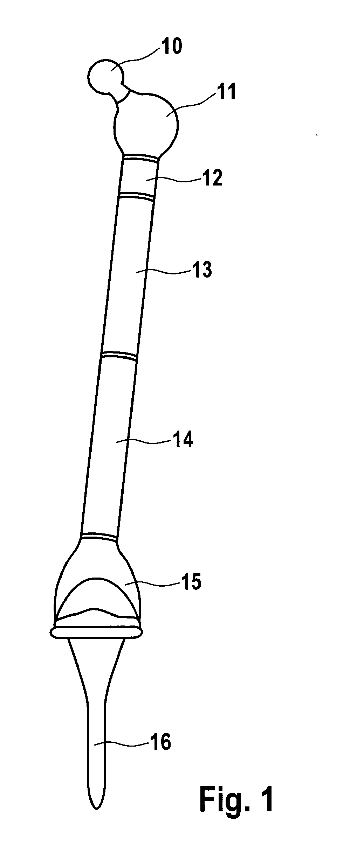

Eine in Fig. 1 gezeigte Endoprothese ist dazu bestimmt, einen von der Hüfte bis unterhalb des Knies reichenden Teil des menschlichen Skeletts zu ersetzen. Ein kugelförmiger Gelenkkopf 10 bildet eine Gelenkfläche, die dazu ausgelegt ist, mit einem Acetabulum zusammenzuwirken. Der Gelenkkopf 10 ist mit einem Kopfstück 11 der Endoprothese über eine Schraubverbindung verbunden. Der den Mittelschaft des Oberschenkelknochens ersetzende Teil der Endoprothese umfasst drei Endoprothesenkomponenten 12, 13, 14. Die Endoprothesenkomponenten 12, 13, 14 sind untereinander und mit dem Kopfstück 11 ebenfalls über Schraubverbindungen verbunden. Ein Kniestück 15 bildet eine gelenkige Verbindung zu einem Schaft 16, der dazu bestimmt ist, die Endoprothese mit einem Unterschenkelknochen zu verbinden.

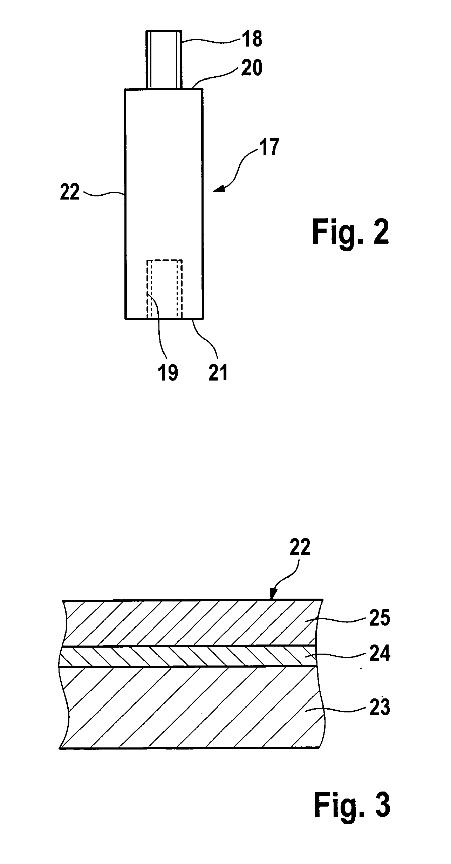

Die Endoprothesenkomponenten 12, 13, 14 sind in unterschiedlichen Längen verfügbar, damit die Endoprothese an unterschiedlich lange Oberschenkelknochen angepasst werden kann. Die Fig. 2 zeigt eine den Endoprothesenkomponenten 12, 13, 14 entsprechende Endoprothesenkomponente 17 in vergrößerter Darstellung. Die Endoprothesenkomponente 17 umfasst einen Schraubbolzen 18 sowie eine gestrichelt angedeutete Schraubbohrung 19. Über den Schraubbolzen 18 und die Schraubbohrung 19 kann die Endoprothesenkomponente 17 an ihren beiden Enden mit gleichartigen Endoprothesenkomponenten, wie etwa den Endoprothesenkomponenten 12, 13, 14, verbunden werden. Der Schraubbolzen 18, die Schraubbohrung 19 sowie die angrenzenden Stirnflächen 20 und 21 bilden also Bereiche an der Oberfläche der Endoprothesenkomponente 17, die dazu ausgelegt sind, mit anderen Endoprothesenkomponenten zusammenzuwirken. Die Mantelfläche 22 der Endoprothesenkomponente 17 hingegen ist dazu ausgelegt, im implantierten Zustand Kontakt mit menschlichem Gewebe einzugehen.

Im Bereich der Mantelfläche 22 ist der die Form der Endoprothesenkomponente 17 vorgebende Körper 23 mit einer Zwischenschicht 24 sowie einer Außenschicht 25 belegt. Die aus Silber bestehende Zwischenschicht 24 belegt den Körper 23 im Bereich der Mantelfläche 22 flächendeckend. Die die Zwischenschicht 24 bedeckende Außenschicht 25 besteht aus Titannitrid mit eingeschlossenen Silberatomen.

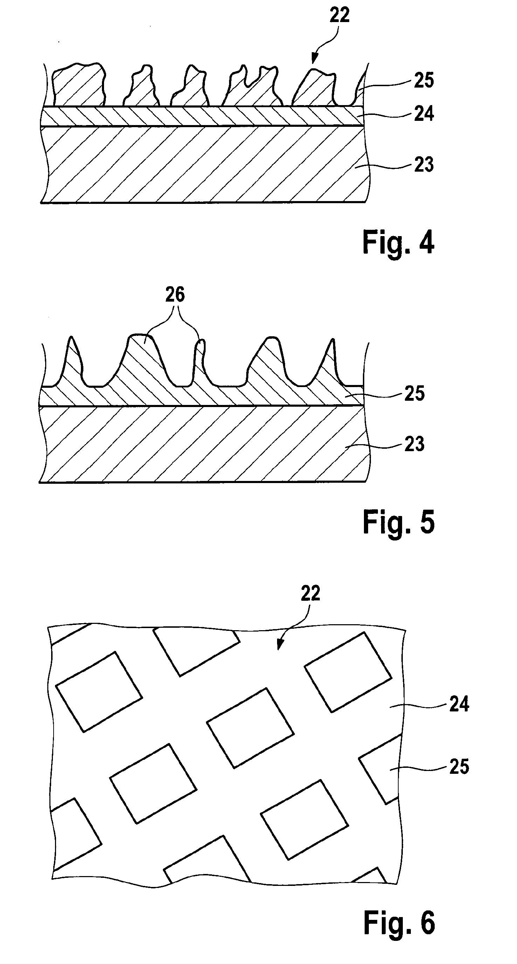

In dem in Fig. 3 gezeigten Ausführungsbeispiel ist die Außenfläche 25 dicht. Das die Mantelfläche 25 umgebende Gewebe kommt also nicht in Kontakt mit der Zwischenschicht 24. Ohne einen Kontakt zum umgebenden Gewebe kann die Zwischenschicht 24 jedoch ihre antimikrobielle Wirkung nicht entfalten. In einem anderen, in Fig. 4 gezeigten Ausführungsbeispiel ist die Außenschicht 25 porös. Die Zwischenschicht 24 kann durch direkten Kontakt mit dem umgebenden Gewebe antimikrobiell wirken.

In dem Ausführungsbeispiel gemäß Fig. 5 ist die Außenschicht 25 unmittelbar auf den die Form der Endoprothesenkomponente 17 vorgebenden Körper 23 aufgebracht. Die Außenschicht 25 hat eine Nanostruktur mit ausgeprägten Spitzen 26. Durch elektrisch leitfähige Spitzen 26 bildet sich ein elektrisches Feld um die Spitzen 26 herum, durch das die antimikrobielle Wirkung der Außenschicht 25 verstärkt wird.

Fig. 6 zeigt eine Draufsicht auf die Mantelfläche 22 der Endoprothesenkomponente 17 in vergrößerter Darstellung. Die Zwischenschicht 24 ist nur inselförmig mit der Außenschicht 25 belegt. Die Außenschicht 25 hat also makroskopische Durchbrechungen, durch die das umgebende Gewebe in direkten Kontakt mit der Zwischenschicht 24 treten kann.

## Patentansprüche

1. Endoprothesenkomponente, umfassend einen die Form der Endoprothesenkomponente (17) vorgebenden Körper (23), bei der der Körper (23) auf Oberflächenanteilen (22), mit denen die Endoprothesenkomponente im implantierten Zustand in Kontakt mit menschlichem Gewebe steht, mit einer Außenschicht (25) belegt ist, die ein Nitrid, ein Oxinitrid oder ein Oxid auf der Basis eines Refraktärmetalls umfasst, **dadurch gekennzeichnet, dass** in der Außenschicht (25) Silber und/oder Kupfer enthalten ist.

2. Endoprothesenkomponente nach Anspruch 1, **dadurch gekennzeichnet, dass** zwischen der Außenschicht (25) und dem Körper (23) eine Zwischenschicht (24) angeordnet ist.

3. Endoprothesenkomponente nach Anspruch 2, **dadurch gekennzeichnet, dass** die Zwischenschicht (24) aus Silber, Kupfer und/oder Gold besteht.

4. Endoprothesenkomponente nach einem der Ansprüche 1 bis 3, **dadurch gekennzeichnet, dass** die Außenschicht (25) durchbrochen ist und dass die Durchbrechungen einen Flächenanteil von mindestens 20%, vorzugsweise mindestens 40%, weiter vorzugsweise mindestens 60% einnehmen.

5. Endoprothesenkomponente nach einem der Ansprüche 1 bis 4, **dadurch gekennzeichnet, dass** die Außenschicht (25) porös ist.

6. Endoprothesenkomponente nach einem der Ansprüche 1 bis 5, **dadurch gekennzeichnet, dass** die Außenschicht (25) eine Mikro- oder Nanostruktur mit ausgeprägten Spitzen (26) hat.

7. Verfahren zum Herstellen einer Endoprothesenkomponente (17) mit den Schritten:
a. Bereitstellen eines die Form der Endoprothesenkomponente vorgebenden Körpers (23);
b. Bereitstellen eines Refraktärmetalls sowie von Silber und/oder Kupfer in Form eines oder mehrerer Targets;
c. Herauslösen von Ionen des Refraktärmetalls sowie von Ionen des Silbers und/oder Kupfers aus dem einen oder den mehreren Targets;
d. Leiten der Ionen zusammen mit einem reaktiven Gas auf einen Oberflächenanteil (22) des Körpers, der für eine Verbindung mit menschlichem Gewebe ausgelegt ist.

8. Verfahren nach Anspruch 7, **dadurch gekennzeichnet, dass** die Ionen mittels Lichtbogen aus dem einen oder der Mehrzahl von Targets herausgelöst werden.

9. Verfahren nach Anspruch 7, **dadurch gekennzeichnet, dass** die Ionen mittels Laser aus dem einen oder der Mehrzahl von Targets herausgelöst werden.

10. Verfahren nach Anspruch 7, **dadurch gekennzeichnet, dass** die Ionen mittels eines Elektronenstrahls aus dem einen oder der Mehrzahl von Targets herausgelöst werden.

11. Verfahren nach einem der Ansprüche 7 bis 9, **dadurch gekennzeichnet, dass** in Schritt a) ein Körper (23) bereitgestellt wird, bei dem für einen Kontakt mit menschlichem Gewebe ausgelegte Oberflächenanteile (22) mit einer Schicht (24) aus Silber, Gold und/oder Kupfer belegt sind.

12. Verfahren nach einem der Ansprüche 7 bis 10, **dadurch gekennzeichnet, dass** in Schritt c) zusätzlich Bruchstücke mit einem Durchmesser von mehr als 20 *µ*m aus dem Target herausgelöst werden und dass die Bruchstücke in Schritt d) auf den Körper (23) geleitet werden.

13. Verfahren nach einem der Ansprüche 7 bis 11, **dadurch gekennzeichnet**, das vor Schritt d) der zu beschichtende Oberflächenanteil des Körpers mit einer Maske aus einem temperaturstabilen Material belegt wird.
